# EUROPEAN PATENT SPECIFICATION

(11) **EP 2 436 034 B1**
(45) Date of publication and mention of the grant of the patent: **19.07.2017**
(21) Application number: 10781127.5
(22) Date of filing: 26.05.2010
(51) Int. Cl.: H01L 27/146, H04N 5/32, A61B 6/03, A61B 6/00

(54) **IMAGING, DATA ACQUISITION, DATA TRANSMISSION, AND DATA DISTRIBUTION METHODS AND SYSTEMS FOR HIGH DATA RATE TOMOGRAPHIC X-RAY SCANNERS**
VERFAHREN UND SYSTEME ZUR BILDGEBUNG, DATENERFASSUNG UND DATENÜBERTRAGUNG FÜR RÖNTGENTOMOGRAPHIESCANNER MIT HOHER DATENRATE
IMAGERIE, ACQUISITION DE DONNÉES D'IMAGE, TRANSMISSION DE DONNÉES, ET PROCÉDÉ ET SYSTÈMES DE DISTRIBUTION DE DONNÉES POUR DES SCANNERS TOMOGRAPHIQUES À RAYONS X À HAUT DÉBIT DE DONNÉES

(30) Priority: 26.05.2009 US 181077 P
(43) Date of publication of application: 04.04.2012
(73) Proprietor: Rapiscan Systems, Inc., Torrance, CA 90503 (US)
(72) Inventor: MORTON, Edward, James, GBN GU1 2SL (GB)
(74) Representative: Wilson, Alan Stuart
(86) International application number: PCT/US2010/036179
(87) International publication number: WO 2010/138572

(56) References cited:
- EP-A1- 1 050 272
- WO-A1-2007/088497
- WO-A1-2008/156764
- US-A- 5 600 303
- US-A1- 2001 038 681
- US-A1- 2002 075 545
- US-A1- 2003 103 212
- US-A1- 2009 060 135
- US-A1- 2009 128 557
- US-B1- 6 417 797

## Description

### BACKGROUND OF THE INVENTION

Applicant has developed a new generation of X-ray systems that implement X-ray sources with more than one electron gun and one or more high voltage anodes within a single vacuum envelope.

The rapid switching of electron guns under electrostatic control enables the fast movement of the effective focal spot of the X-ray tube and the rapid generation of sets of tomographic X-ray scan data without the use of moving parts. By configuring the firing sequence of the electron guns appropriately, an optimal set of X-ray projection data can be collected at rates far higher than in conventional systems.

Accordingly, because Applicant's latest generation X-ray tomography systems are capable of generating very high resolution tomography X-ray data at high speeds, a number of new data acquisition and data transmission and distribution systems are enabled, and/or required, to take full advantage of, and/or optimally manage, these high data rate systems. For example, current storage systems and data transmission networks are unable to effectively transfer high volumes of image data to network of operator workstations without undue delay. Moreover, such delay increases as the number of image generating systems on a network increases and as the number of operators required to analyze images also increases.

Therefore, improved data acquisition, transmission and distribution systems are needed to take full advantage of, and/or optimally manage, high resolution X-ray data generated at high speeds, such as data rates greater than 1 Gbit/second.

EP1050272A1 discloses increasing the rotational speed of a gantry in an imaging system to improve resolution but it does not address the problem of how to control the rotational speed of the gantry during a scan.

### SUMMARY OF THE INVENTION

The present invention provides an X-ray system according to the appended claims.

### BRIEF DESCRIPTION OF THE DRAWINGS

Figure 1 is a block diagram of one embodiment of the X-ray system of the present invention;
Figure 2 depicts an imaging area relative to the object being inspected;
Figure 3 depicts an exemplary sinogram generated by the present invention;
Figure 4 depicts a variable scan, based on the object being scanned;
Figure 5 is an exemplary diagram of variable pixelation levels of an object being scanned;
Figure 6 is a diagram of a collection of X-ray systems with improved data transmission; and
Figure 7 depicts exemplary two-dimensional images derived from a full three-dimensional data set.

### DETAILED DESCRIPTION OF THE INVENTION

The X-ray systems disclosed in the above-mentioned applications, which are herein incorporated by reference, enable the rapid and real-time control of X-ray source trajectories. The present invention takes advantage of this capability by dynamically controlling X-ray source trajectories and dynamically altering the image reconstruction methods based on both the properties of the object under investigation and the quality of the inspection decision that is required. This dynamic system optimization can have several benefits, including reduction of scan dose, reduction of computation complexity and reduction of data bandwidth. Other objectives can be addressed using the same and related optimization methods.

The following disclosure is provided in order to enable a person having ordinary skill in the art to practice the invention. Exemplary embodiments are provided only for illustrative purposes and various modifications will be readily apparent to persons skilled in the art. The general principles defined herein may be applied to other embodiments and applications without departing from the spirit and scope of the invention. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purpose of clarity, details relating to technical material that is known in the technical fields related to the invention have not been described in detail so as not to unnecessarily obscure the present invention.

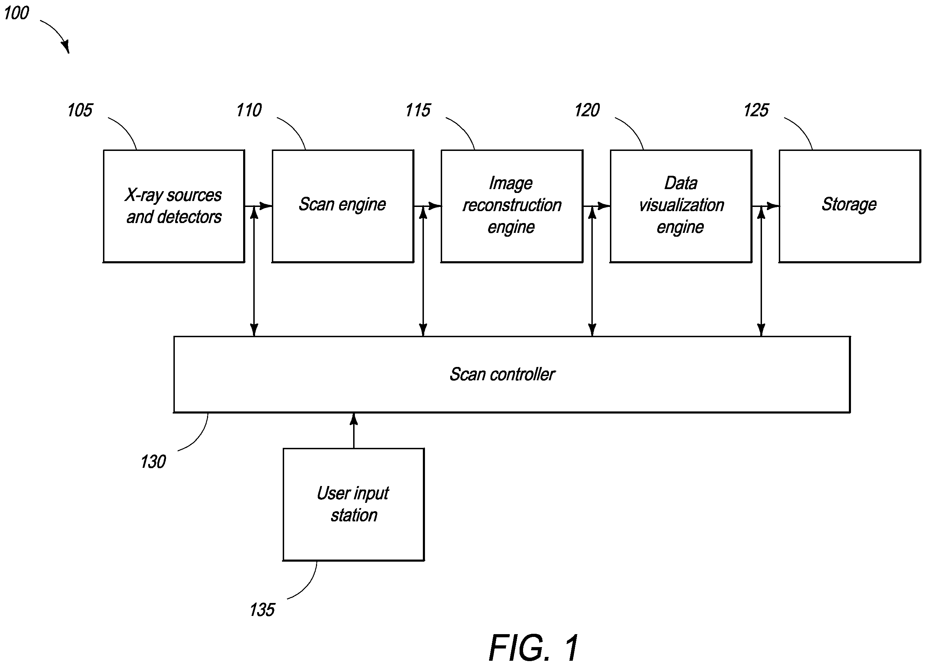

The present invention will now be discussed in context of embodiments as illustrated in the accompanying drawings. Referring to Figure 1, one embodiment of an X-ray system configuration is shown. The X-ray system 100 comprises a set of X-ray sources and X-ray detectors 105 which are capable of collecting information about the object under inspection. The set of sources and detectors (sensors) 105, are, in one embodiment configured to generate transmitted X-ray data set suitable for tomographic image reconstruction. Alternatively, in another embodiment, the set of sources and sensors 105 are configured to generate a set of diffraction X-ray data or a set of backscattered X-ray data. Alternatively, in yet another embodiment, sources and sensors 105 are configured to generate a set of stereoscopic transmission X-ray data. One of ordinary skill in the art would appreciate that the present invention can be applied to other source and sensor geometries.

As shown in Figure 1, a scan engine 110 coordinates scanning of the object under inspection and data collection from the X-ray sources and detectors 105. The scan engine 110 is programmable on a scan-by-scan basis and also within a scan to allow rapid re-optimization of the X-ray system 100.

As shown in Figure 1, an image reconstruction engine 115 takes the raw data and/or calibrated data which is output from the scan engine 110 and converts this data into a recognizable set of image data or processed data. It should be appreciated that calibrated data can be generated from a pre-processor which is part of the scan engine 110 and which can also be considered the first stage of the image reconstruction engine 115.

For example, suppose that the scan engine 110 normally provides a set of calibrated transmission X-ray projection data. The image reconstruction engine 115 takes this projection data and converts this data into an X-ray image using a plurality of processing techniques such as filtered back projection to convert the projection data back into a 2- or 3-dimensional tomographic X-ray image. As used herein, raw data refers to data directly transferred from one or more detectors or detector data that has not yet been subject to calibration or normalization. Raw data which has been subject to calibration or normalization is referred to as calibrated data. Processed data or image data is calibrated data that has been passed through one or more filters.

Processed data from the image reconstruction engine 115 is optionally passed to an optional data visualization engine 120. The data visualisation engine 120 takes the reconstructed image data and renders it to a human readable screen. This data visualization engine 120, in one embodiment, is capable of performing functions such as interpolation, three-dimensional projection into a two-dimensional image, and image coloring based on reconstructed image density, materials type or other parameters as required.

In one embodiment, a storage device 125, such as a RAID array, set of hard disks, or other memory systems, is provided for archiving X-ray image data. It will be understood by an expert that the storage device 125 shall be capable of storing data from any point in the signal chain, and not just at the output of the data visualisation engine.

The scan controller 130 is capable of interpreting X-ray image data at the output from all components in the imaging chain. The scan controller 130 is also capable of outputting control information to all components in the imaging chain. In this way, the scan controller 130 has the ability to optimize the entire system operation.

The scan controller 130 also receives input data from a user input station 135, in which users can input their constraints on the image optimization process via, for example, a touch screen, mouse, or keyboard in communication with a computing device. For example, in a security screening system the user may select a current threat level (e.g. High, Medium or Low). In a medical system, the user may select a dose or spatial resolution constraint (e.g. High Diagnostic Quality, Low Dose).

The scan controller 130 thus takes input from two different sources, namely user constraints and X-ray image data, and implements a method which processes the X-ray image data according to the user constraints in order to continually and dynamically re-optimize the operation of the X-ray sources and detectors 105, the scan engine 110, the image reconstruction engine 115, and/or the data visualisation engine 120.

It should be appreciated that the functions and features of the X-ray sources and detectors 105, scan engine 110, image reconstruction engine 115, and/or data visualization engine 120, as described herein, are enabled by hardware, such as servers comprising at least one processor, executing a plurality of programmatic instructions which are stored in a memory, such as RAM, ROM, hard disk, or other memory structures. The various engines may be integrated into one or more servers which are remote or local to one another. The components of the X-ray system 100 may therefore be local or remote from each other, may be integrated into a single server or distributed among a plurality of servers across a network, and/or may communicate with each other through any wired or wireless form of transmission and reception. It should further be appreciated that each engine comprises at least one data receiver, data transmitter, and data processor capable of executing a plurality of programmatic instructions, stored in memory, to perform the functions and features of the engines, as described herein.

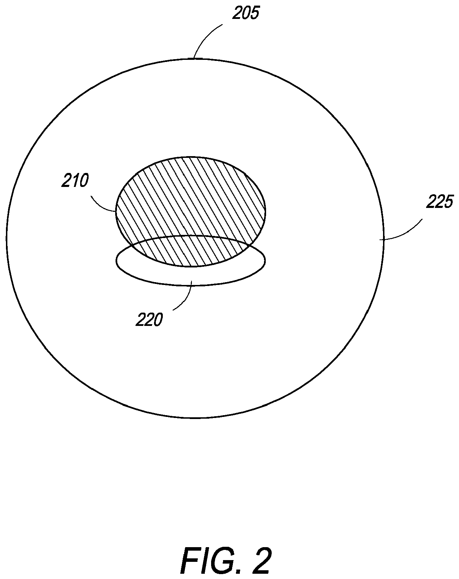

Referring to Figure 2, in one embodiment, the tomographic X-ray imaging system of the present invention images an object 210 which partially fills the imaging area 205. Conventionally, a rotating gantry X-ray source is used and projection data is accumulated for all projection angles and from all detector elements. It is computationally expensive to collect and process all of this data when only a fraction of the X-ray inspection area is actually filled with a relevant object. The fraction of the X-ray inspection area which is filled with air 225 or the object support 220 is generally of little interest to the user.

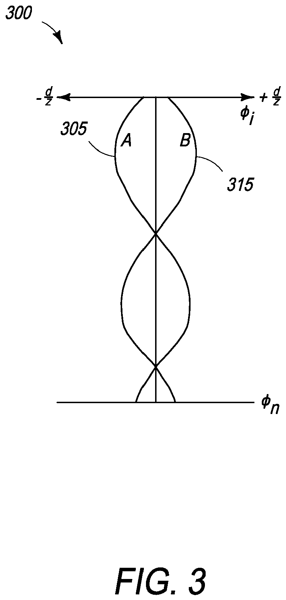

In one embodiment, this scanning scenario can be improved in the following manner. Rather than scan and process the entire imaging area 205, raw scan data generated by the X-ray system is first analyzed by the scan controller 130. Regions of the image data that contain relevant information are differentiated from those areas which contain no relevant data. As shown in Figure 3, a sinogram chart 300 can be used to review the data. The chart 300 arranges data from each detector in a horizontal row (from -d/2 through 0 to +d/2 where d = total number of detectors in a projection) and data from each source rotation angle (from Phi_1 to Phi_n where n = number of projection angles) in consecutive rows to form a two dimensional data set. The trajectory thorough the sinogram data set for two points in the object, labelled A 305 and B 315, is also shown in the diagram.

In the present invention, once the first line of the sinogram (Phi_1) has been collected, it is known, *a priori,* what part of the sinogram will need to be collected at all other projections angles. The scan controller can therefore optimize the scan engine to only collect and process data from those detector elements which are actually needed, e.g. would actually generate relevant data, thereby saving on front end data bandwidth and computer power.

Mathematically, the sinogram data is linked forward to the backprojected image by the Radon Transform. Under this transform, each point in the sinogram represents one backprojected line through the reconstructed image. Since it is known which parts of the sinogram contain useful data, and hence were collected by the scan engine, the image reconstruction engine is therefore constrained to only filter and backproject the valuable parts of the sinogram data. This can yield major savings in computational cost in the image reconstruction engine.

The image reconstruction engine is typically designed to permit full image reconstruction of the whole image reconstruction circle at full reconstructed image slice rate. Very often, only a fraction of the image reconstruction circle has an object therein, and systems designed with the use of the scan controller can be provided with a much lower cost image reconstruction engine that is optimized to dynamically reconstruct only the relevant part of the object. Preferably, the scan engine and image reconstruction engine shall comprise, or have access to, a buffer memory to allow sequences of images where the object is larger than the designed image reconstruction circle fill capacity to be stored temporarily until the image reconstruction engine has caught up with the current data flow.

The data visualisation engine is provided with the coordinates (such as slice number and position within the slice) of the sub-image which is provided by the image reconstruction engine and it uses these coordinates (slice number and position within the slice) to reference the reconstructed image data to the appropriate part of the three-dimensional reconstructed image volume prior to data visualization. The net effect is a substantial reduction in data bandwidth to the storage system. This simplifies design of network attached storage and reduces the cost of storage solutions since unwanted background data is not stored.

In a further example of the use of this invention, it may be the case that the object to be inspected has variable inspection significance along its length. For example, some parts of the human body are quite symmetric in the scan direction (the legs) and here it may be advantageous to design a tomographic image system which generates a lower spatial resolution in the scan direction (along the legs) compared to that in the scan plane.

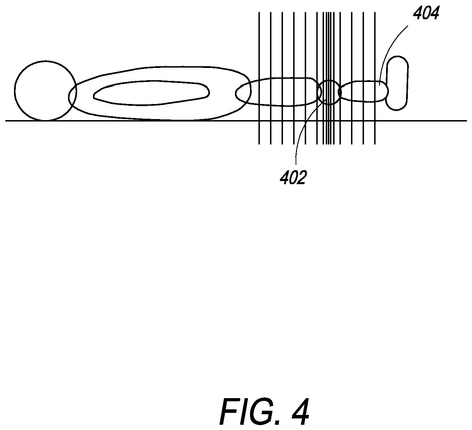

To do this, the user may request the scan controller to use a low dose scanning method, which could be achieved by dynamically controlling the scan rotation rate to allow a degree of scan-direction partial volume artifact when imaging the long bones of the leg in order to reduce dose. Referring to Figure 4, when arriving at the knees 402 which are substantially variable in the scan direction, the scan controller can automatically direct the scan engine to increase scan rate (and hence also X-ray dose) in order to create a high-resolution image of the knees 402 prior to returning to a lower dose rate once back to the long bones 404 past the knee.

Mathematically, the scan controller can determine the rate of change of information in the scan direction by subtracting each new sinogram from the previous sinogram and the difference is an indication of the rate of change of information in the scan direction.

In a further embodiment of this invention, it is possible to use the scan controller to adjust, on a dynamic basis, the spatial resolution in the data collected by the scan engine. This data is then passed through to the image reconstruction engine which in turn provides a variable resolution image. The reconstructed image is encoded for display by the data visualization engine so that the image is represented correctly in both the spatial and time domains.

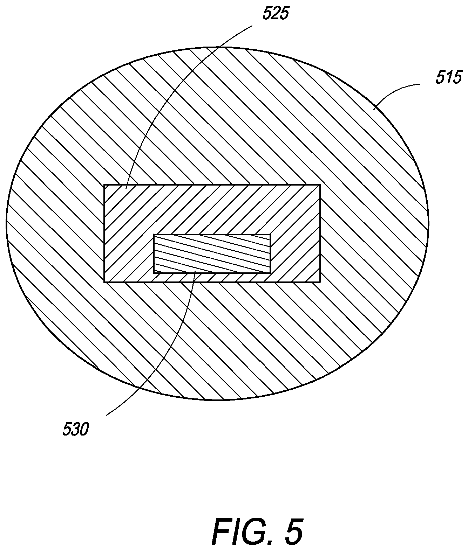

As an example, in a security inspection application providing three-dimensional tomographic image reconstruction, one can reconstruct low density regions, such as clothes, with coarse pixelation 515 (such as greater than ten times the smallest pixel size), reserving medium granularity pixelation 525 (such as 2-10 times the smallest pixel size) and high granularity pixelation 530 (such as the smallest pixel size, i.e. 1mm x 1mm) for complex objects, such as a lap-top computer, which resembles a higher-threat object, as shown in Figure 5. By relaxing the resolution requirements in some areas, and thus requiring less computation, the spare computation capacity can be allocated to provide enhanced resolution in complex areas of the image. The net effect is a reduction in data bandwidth, a reduction in computational cost and a reduction in storage requirements with little detriment to diagnostic quality.

In one embodiment, the sinogram data directs the scan controller regarding what computational power should be allocated in order to form the reconstructed image. Sinogram regions that exhibit a high degree of variation, such as those regions with a smaller signal to noise ratio (i.e. a ratio of less than 20, preferably less than 10), are linked directly to those areas of the reconstructed image where a high image resolution should be achieved. A high image resolution is typically less than 2mm x 2mm x 2mm in terms of pixel size, while a low image resolution is typically greater than 5mm x 5mm x 5mm in terms of pixel size. Regions of the sinogram that exhibit a low degree of variation, such as those regions with a higher signal to noise ration (i.e. a ratio of greater than 40, preferably greater than 50), are linked to those areas of the reconstructed image where a low image resolution can be tolerated.

One of ordinary skill in the art would appreciate that the variable resolution reconstructed image should be encoded such that the data visualization engine can reference the variable resolution reconstructed image data into the appropriate locations in the three-dimensional data set for human display purposes. It is further understood that suitable image processing methods, such as three-dimensional image interpolation, can be used to render an image with uniformly high spatial resolution to the screen even though the underlying data may have been reconstructed at variable resolution.

In a further embodiment of this invention, it is recognized that the nature of the image reconstruction process itself can be affected by the information that is being processed by the scan engine. By way of example, consider an imaging system which is typically looking for low atomic number, low contrast objects in a uniform matrix. Such an image is suited to a filtered backprojection tomographic image reconstruction approach. Suppose that there is, from time to time, a contaminant that is of high atomic number which enters the imaging volume. In this case, the high atomic number material is likely to result in unwanted beam hardening artifact which can distort the analysis of the low atomic number materials. There are known iterative processes which are better at minimizing the effect of beam hardening than typical filtered backprojection algorithms. In this case, the scan controller can inspect the sinogram for particularly highly attenuating objects and can substitute iterative algorithms for those regions where the highly attenuating objects are located, and can apply the less computationally demanding filtered backprojection algorithm to all other areas of the image. The result is a minimization in beam hardening artifact and an improvement in ultimate image analysis.

It shall be evident to one skilled in the art that it is possible to apply the methods described here simultaneously and individually depending on the optimization requirements that have been set by the user, on the cost constraints that were evaluated at the time of system manufacture, and on the availability and performance of storage systems.

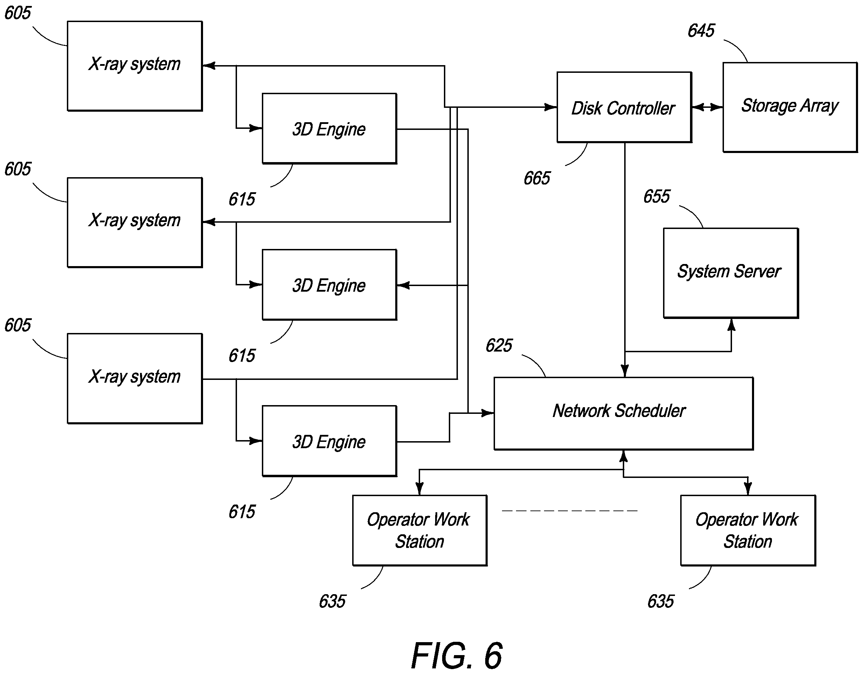

As discussed above, the high data rate, high throughput system requires an improved image transmission system to provide a responsive, scalable and cost effective transmission method. Referring to Figure 6, a collection of X-ray systems 605, each capable of generating high volumes of data where each data set comprises a three-dimensional image of an object, is shown. Each X-ray system 605 is provided with a high speed dedicated network link which can take this three-dimensional data set directly to a high capacity storage array 645 at full bandwidth with little latency.

The database on the storage array 645 is managed by a system server 655 which is connected to the X-ray systems 605 and to the disk controller 665 via a network. A series of operators view three-dimensional image data at a series of networked workstations 635.

A job dispatcher application executes on the system server 655 and recognizes when new three-dimensional data is available. The job dispatcher application allocates a particular operator to review the new three-dimensional data set and the operator reports his or her findings back to the system server 655 via the network. At this point, the three-dimensional data set may be discarded, or it may be archived for medium term or long term storage.

Such a system can be very slow and use very large amounts of network bandwidth due to the size of the three-dimensional data sets. In the present invention, a 3D engine component 615 is coupled to the system, one 3D engine 615 per X-ray system 605. The 3D engine 615 processes the full three-dimensional data set that is generated by the X-ray system 605 in real time since it is directly connected to the X-Ray system 605 rather than being constrained by the bandwidth and latency of an intermediate network.

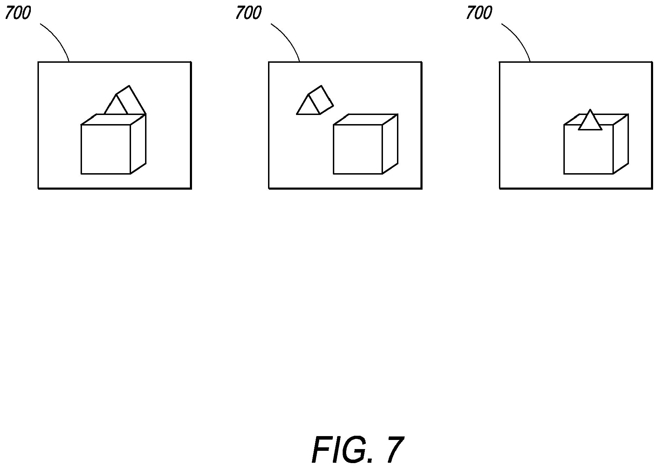

The 3D engine 615 processes the X-ray system 605 data into one or more two-dimensional images which represent projections through the three-dimensional data. Example two-dimensional images 700 that are derived from a full three-dimensional data set are provided in Figure 7. The images 700 depict a three-dimensional triangular object and a cube like object in various orientations. It shall be evident to one skilled in the art that the size of the two-dimensional image is small compared to that of the full three-dimensional data set. Therefore, the network transmission time for the 2D image is short compared to that for the full 3D data set.

In the present invention, the 3D engine 615 output connects directly to the network such that the job dispatcher application running on the system server 655 can first dispatch a set of compact 2D images to an operator workstation 635 for immediate review. As a minimum, one 2D image will be rapidly generated and dispatched to the operator. More typically, 5 to 8 2D images will be pre-rendered and dispatched to an operator workstation 635. Very frequently, the operator can inspect the object with sufficient integrity that they do not need access to the full three-dimensional data set, thereby permitting the operator workstation 635 to be of a relatively low specification and reducing its cost compared to a full 3D workstation. The network can be almost any commercially available network of moderate bandwidth, such as a gigabit ethernet network.

In the event that the inspector can not inspect the object using 2D images only, the operator can signal to the job dispatcher that inspection is incomplete and that the image data should be transferred to a fully 3D operator workstation with a suitably trained operator for further review.

In this case, the job dispatcher will locate a suitably qualified 3D operator and will first send the set of 2D images to the operator for review. At this point, the operator will start to analyze the image data from 2D images only. In the background, the job dispatcher will start to transfer the full 3D data set to the 3D operator. Once the 3D data set has arrived at the operator workstation, the 3D engine inside the operator workstation will prepare the data set for 3D review by the operator. When the 3D data set is ready for viewing, the image workstation enables a set of three-dimensional image analysis tools and the operator can start to make use of these in an interactive fashion. The operator thus transfers from a 2D inspection mode to a 3D inspection mode as the necessary data becomes available.

Such a two-stage image transmission approach minimizes the time taken for the operator to start work but still provides all the data for review when needed. This is achieved using networks of modest bandwidth and latency with a consequently low cost.

In another embodiment, a network of operators, all of whom are qualified to inspect 3D data and all of whom have access to a 3D operator workstation, is provided. In this case, the operator will receive 2D data immediately, and can request full 3D image data when he or she is unable to complete his or her inspection using 2D pre-rendered images only. Again this allows a high reliability low cost network to be utilized while maintaining high inspection throughput.

While the exemplary embodiments of the present invention are described and illustrated herein, it will be appreciated that they are merely illustrative. It will be understood by those skilled in the art that various changes in form and detail may be made therein without departing from or offending the scope of the appended claims.

## Claims

1. An X-ray system comprising:
a source of radiation having a scan rotation ;
a plurality of detectors arranged to generate raw X-ray data;
a scan engine (110) coupled to the plurality of detectors and arranged to receive the raw X-ray data and generate calibrated X-ray data;
an image reconstruction engine (115) coupled to the scan engine (110) and arranged to convert the calibrated X-ray data into one or more processed X-ray images; and
**characterised by**
a scan controller (130) coupled with said plurality of detectors and at least one of said scan engine (110) or image reconstruction engine (115), wherein said scan controller (130) is arranged to receive said raw X-ray data, generate sinogram data to determine a rate of change of information in a scan direction and issue instructions to said source of radiation to control a rotation rate of said source of radiation based on the determined rate of change of information.

2. The X-ray system of claim 1 wherein the scan controller (130) issues instructions to said scan engine (110) to modify a spatial resolution of a scan based upon said raw X-ray data.

3. The X-ray system of claim 2 wherein said spatial resolution is modified by generating a first spatial resolution in a scan direction and a second spatial resolution in a scan plane, the first spatial resolution being different than the second spatial resolution.

4. The X-ray system of claim 1 wherein the scan controller (130) issues instructions to said scan engine (110) to generate a first resolution in a scan and a second resolution in the scan wherein said first resolution and said second resolution are different.

5. The X-ray system of claim 4 wherein said scan engine transmits said scan having the first resolution and the second resolution to the image reconstruction engine (115).

6. The X-ray system of claim 5 wherein said image reconstruction engine (115) generates image data having variable resolution based on said scan.

7. The X-ray system of claim 4 wherein said image reconstruction engine (115) generates image data having at least one area of coarse pixelation, medium granularity pixelation, or high granularity pixelation.

## Patentansprüche

1. Röntgensystem, umfassend:
eine Strahlungsquelle mit einer Scan-Rotation;
eine Vielzahl von Detektoren, die angeordnet sind, um Röntgen-Rohdaten zu generieren;
eine Scan-Engine (110), die an die Vielzahl von Detektoren gekoppelt ist und angeordnet ist, um die Röntgen-Rohdaten zu empfangen und kalibrierte Röntgendaten zu generieren;
eine Bildrekonstruktions-Engine (115), die an die Scan-Engine (110) gekoppelt ist und angeordnet ist, um die kalibrierten Röntgendaten in ein oder mehrere verarbeitete Röntgenbilder umzuwandeln;
und **gekennzeichnet durch**
eine Scan-Steuerung (130), die mit der Vielzahl von Detektoren und mindestens einer von der Scan-Engine (110) oder Bildrekonstruktions-Engine (115) gekoppelt ist, wobei die Scan-Steuerung (130) angeordnet ist, um die Röntgen-Rohdaten zu empfangen, Sinogrammdaten zu generieren, um eine Rate der Informationsänderung in einer Scan-Richtung zu ermitteln und Anweisungen an die Strahlungsquelle auszugeben, eine Rotationsrate der Strahlungsquelle basierend auf der ermittelten Rate der Informationsänderung zu steuern.

2. Röntgensystem nach Anspruch 1, wobei die Scan-Steuerung (130) Anweisungen an die Scan-Engine (110) ausgibt, eine räumliche Auflösung eines Scans basierend auf den Röntgen-Rohdaten zu modifizieren.

3. Röntgensystem nach Anspruch 2, wobei die räumliche Auflösung modifiziert wird, indem eine erste räumliche Auflösung in einer Scan-Richtung und eine zweite räumliche Auflösung in einer Scan-Ebene generiert werden, wobei sich die erste räumliche Auflösung von der zweiten räumlichen Auflösung unterscheidet.

4. Röntgensystem nach Anspruch 1, wobei die Scan-Steuerung (130) Anweisungen an die Scan-Engine (110) ausgibt, eine erste Auflösung in einem Scan und eine zweite Auflösung in dem Scan zu generieren, wobei die erste Auflösung und die zweite Auflösung unterschiedlich sind.

5. Röntgensystem nach Anspruch 4, wobei die Scan-Engine den Scan mit der ersten Auflösung und der zweiten Auflösung an die Bildrekonstruktions-Engine (115) überträgt.

6. Röntgensystem nach Anspruch 5, wobei die Bildrekonstruktions-Engine (115) Bilddaten mit variabler Auflösung basierend auf dem Scan generiert.

7. Röntgensystem nach Anspruch 4, wobei die Bildrekonstruktions-Engine (115) Bilddaten mit mindestens einem Bereich mit grober Pixelung, Pixelung mit mittlerer Körnigkeit oder Pixelung mit hoher Körnigkeit generiert.

## Revendications

1. Système à rayons X comprenant :
une source de rayonnement ayant une rotation de balayage ;
une pluralité de détecteurs conçus pour générer des données brutes de rayons X ;
un moteur de balayage (110) couplé à la pluralité de détecteurs et conçu pour recevoir les données brutes de rayons X et générer des données de rayons X étalonnées ;
un moteur de reconstruction d'image (115) couplé au moteur de balayage (110) et conçu pour convertir les données de rayons X étalonnées en une ou plusieurs images de rayons X traitées ;
et **caractérisé par** :
un dispositif de commande de balayage (130) couplé à ladite pluralité de détecteurs et audit moteur de balayage (110) et/ou audit moteur de reconstruction d'image (115), dans lequel ledit dispositif de commande de balayage (130) est conçu pour recevoir lesdites données brutes de rayons X, générer des données de sinogramme pour déterminer une vitesse de changement d'informations dans une direction de balayage et
délivrer des instructions à ladite source de rayonnement pour réguler une vitesse de rotation de ladite source de rayonnement en se basant sur la vitesse déterminée de changement d'informations.

2. Système à rayons X selon la revendication 1, dans lequel le dispositif de commande de balayage (130) délivre des instructions audit moteur de balayage (110) pour modifier une résolution spatiale d'un balayage en se basant sur lesdites données brutes de rayons X.

3. Système à rayons X selon la revendication 2, dans lequel ladite résolution spatiale est modifiée en générant une première résolution spatiale dans une direction de balayage et une seconde résolution spatiale dans un plan de balayage, la première résolution spatiale étant différente de la seconde résolution spatiale.

4. Système à rayons X selon la revendication 1, dans lequel le dispositif de commande de balayage (130) délivre des instructions audit moteur de balayage (110) pour générer une première résolution dans un balayage et une seconde résolution dans le balayage, dans lequel ladite première résolution et ladite seconde résolution sont différentes.

5. Système à rayons X selon la revendication 4, dans lequel ledit moteur de balayage transmet ledit balayage ayant la première résolution et la seconde résolution au moteur de reconstruction d'image (115).

6. Système à rayons X selon la revendication 5, dans lequel ledit moteur de reconstruction d'image (115) génère des données d'image ayant une résolution variable en se basant sur ledit balayage.

7. Système à rayons X selon la revendication 4, dans lequel ledit moteur de reconstruction d'image (115) génère des données d'image ayant au moins une zone de pixellisation grossière, de pixellisation à granularité moyenne ou de pixellisation à forte granularité.
